# NEW EUROPEAN PATENT SPECIFICATION

(11) **EP 0 264 434 B2**
(45) Date of publication and mention of the opposition decision: **01.02.2006**
(45) Mention of the grant of the patent: 13.04.1994
(21) Application number: 87903197.9
(22) Date of filing: 01.05.1987
(51) Int. Cl.: C12P 21/08, G01N 33/569, C12Q 1/68

(54) **DETECTION OF A UNIQUE CHLAMYDIA STRAIN ASSOCIATED WITH ACUTE RESPIRATORY DISEASE**
NACHWEIS EINES MIT ATMUNGSKRANKHEITEN VERBUNDENEN EINZELSTAMMES VON CHLAMYDIA
DETECTION D'UNE SOUCHE UNIQUE DE CHLAMYDIA ASSOCIEE A DES MALADIES RESPIRATOIRES AIGUES

(30) Priority: 01.05.1986 US 858380
(43) Date of publication of application: 27.04.1988
(60) Divisional application: 93110955.7
(73) Proprietor: Washington Research Foundation, Seattle, Washington 98102 (US)
(72) Inventor: GRAYSTON, J., Thomas, Seattle, WA 98105 (US); KUO, Cho-chou, Seattle, WA 98115 (US); WANG, San-pin, Redmond, WA 98052 (US); CAMPBELL, Lee, Ann, Seattle, WA 98155 (US)
(74) Representative: Bizley, Richard Edward
(86) International application number: PCT/US1987/001011
(87) International publication number: WO 1987/006617

(56) References cited:
- EP-A- 0 124 124
- WO-A-85/02685
- WO-A-86/02355
- GB-A- 2 156 074
- Stephens et al., J. Immunol., Vol. 128 (1982), 1083-1089
- Caldwell et al., Infect. Immun. (May 1984), 306-314
- Saikku et al. J. Infect. Dis., Vol. 151 (May 1985, 832-839)
- Darougar et al., Br. J. Vener. Dis. 56, (1980), 404-407
- Campbell et al., Infect. Immun. (Jan. 1990), 93-97 (as an expert opinion)
- Fukushi et al., Int. J.Systematic Bacteriol. 42 (1992), 306-308 (as an expert opinion)
- J. Thomas Grayston; Immunization Against Trachoma; First International Conference on Vaccine Against Viral and Rickettsial Diseases in Man; PAHO Scientific Publication 147: 546-9, 19657
- R. St. C. Dwyer et al.; Chlamydia Infection: Results of Micro-Immunoflourescence Tests for the Detection of Type-specific Antibody in Certain Chlamydia Infections; Brit. J. Vener. Dis. (1972) 48, pp. 452-459
- P. Saikku et al.; Poster presented at the 5th International Symposium on Human Chlamydia Infections, Lund, 15-19 June, 1982; Serological Studies in Chlamydial Pneumonias
- Terho et al. (1982), In "Chlamydial Infections" (Mardh eds.), Elsevier Biomedical Press, pages 321-328
- Persson et al. (1982), In Chlamydial Infections" (Mardh eds.) Elsevier Biomedical Press, pages 325-328.
- Tam et al. (1982), in "Chlamydial Infections" (Mardh eds.), Elsevier Biomedical Press, pages 317-320.
- Stephens et al. (1982), In "Chlamydial Infections" (Mardh eds.), Elsevier Biomedical Press, pages 329-332.
- Fuentes et al. (1985). Immunol Lett Vol. 10, No. 6, pages 325-327.
- Treharne & Forsey (1983), British Medical Bulletin, Vol. 39, No. 2, pages 194-200.
- Evans & Woodland (1983), British Medical Bulletin, Vol. 39, No. 2, pages 181-186.
- Grayston et al. (1989), Int. J. Sys. Bact., Vol. 39, No. 1, pages 88-90
- Burney et al., Intl. J. Epidemiol, 13:491 (1984)
- Grayston et al., Eur. J. Clin. Microbial. Infect. Dis., 8:191-202 (1989)
- S.-P. Wang & J. T. Graystone Am. J. of Ophthalmology, Vol. 70, No.3, p. 367-374 (1970)
- Abstracts of the 1984 ICAAC (1984)
- J. Clin. Microbiol., 26(6), 1034-1037 (1986)
- J. Gen. Microbiol., 137, 465-475 (1991)
- J. Clin. Microbiol., 29(6), 1188-1193 (1991)
- ATCC, Animal Viruses and Antisera, Chlamydiae and Rickettsiae, 7th ed. (1996), 187/188
- J. Clin. Microbiol., 35(3), 620-623 (1997)
- Programm and Abstracts of the 32nd ICAAC, No. 542, (1992)

## Description

### Technical Field

The present invention relates to the detection of Chlamydia strains in general, and more specifically, to the use of monoclonal antibodies in the detection of a unique Chlamydia strain associated with respiratory infection.

### Background Art

Chlamydia are obligate intracellular parasites that are ubiquitous throughout the animal kingdom and are known to cause a variety of diseases. The Chlamydia genus has two recognized species, C. trachomatis and C. psittaci. with further differentiation of C. trachomatis into three biovars. C. trachomatis, the species that is a primary human pathogen, causes ocular, respiratory, and urogenital infections. C. trachomatis can initially colonize the mucous membranes, including the conjunctiva, nasopharynx, rectum, urethra, and cervix, after which the infection may spread to affect the deeper organ systems.

The other species of Chlamydia, C. psittaci, is responsible for the human respiratory disease, psittacosis. Psittacosis is usually contracted by exposure to infected birds, outbreaks of psittacosis having occurred in association with turkey and duck processing plants and among pet shop clientele. The disease often resembles atypical pneumonia and may range in severity from mild to fatal. Although epidemics of such pneumonia have usually been traced to infected birds, an avian vector is not always found. For instance, during a radiographic survey in northern Finland in 1978, an epidemic of mild pneumonia was found in two communities; however, avian transmission was not detected.

Seroepidemiological studies have implicated an unusual strain of Chlamydia in this and several other pneumonia epidemics in northern Europe. This new strain of Chlamydia has more recently been established as an important respiratory pathogen, being responsible for perhaps up to 12% of the atypical pneumonia cases in adult populations studied. However, most infections have been inadequately treated with antibiotics due to a mistaken belief that the causative agent was Mycoplasma pneumoniae or influenza virus A or B. While antibiotic treatment with erythromycin in the low doses used for M. pneumoniae is effective against Mycoplasma infection, erythromycin at this dose is not effective against chlamydial infections. Further, these infections are often characterized by a relapsing illness after an apparent resolution of symptoms.

In light of this association between a new strain of Chlamydia and acute respiratory disease, it would be highly desirable to provide an accurate and cost-effective method for diagnosing the presence of this new strain, thereby permitting an effective antibiotic treatment of the primary infection. The present invention provides such a method and is further characterized by other related advantages.

### Disclosure of the invention

Briefly stated, the present invention provides methods for detecting the presence of a TWAR strain of Chlamydia (hereinafter referred to as the TWAR organism) in biological samples. These methods utilize a monoclonal antibody directed against an antigenic determinant of the TWAR organism specific for the TWAR organism.

In a first aspect, the invention provides claim 1.

In one aspect of the invention, the method comprises the steps of (a) exposing a biological sample suspected of containing the TWAR stain defined above to a labeled monoclonal antibody directed against an antigenic determinant of the TWAR organism: and (b) detecting the presence of immunocomplexes formed between the monoclonal and the TWAR strain defined above.

A related method for detecting the presence of the TWAR strain defined above comprises (a) combining a biological sample suspected of containing the TWAR strain defined above with HeLa cells such that the TWAR strain defined above becomes intracellular; (b) incubating the HeLa cells containing the TWAR strain defined above ; (c) exposing the HeLa cells/TWAR strain defined above to a labeled monoclonal antibody directed against an antigenic determinant of the TWAR strain defined above; and (d) detecting the presence of immunocomplexes formed between the TWAR strain defined above and the labeled monoclonal antibody. In a particularly preferred embodiment of the present invention, the step of combining comprises inoculating the HeLa cells with the sample and subsequently centrifuging the Inoculated sample onto the HeLa cells. It is also preferable to pretreat the HeLa cells with DEAE-dextran and cyclohexamide.

A further aspect of the present invention discloses a method for determining the presence of antibodies to the TWAR strain defined above Chlamydia in a biological sample. The method generally comprises (a) incubating the biological sample with elementary bodies of said TWAR organism to form a reaction mixture; and (b) analyzing the reaction mixture to determine the presence of antibodies to the TWAR. stain defined above in a preferred embodiment, the step of analyzing comprises contacting the reaction mixture with a labeled specific binding partner for the antibody and detecting the label. In this regard, the specific binding partner may be goat anti-human IgM or goat anti-human IgG.

In all of the methods briefly described above, the monoclonal antibody may be labeled with an enzyme, fluorophore, radioisotope, or luminescer. In this regard, the step of detection would normally be by enzyme reaction, fluorescence, radioactivity, or luminescence emission, respectively.

The methods described above may be applied to a variety of biological samples In order to detect the presence of the TWAR organism or antibodies to the TWAR organism. Bodily secretions, bodily fluids, and tissue specimens are all suitable samples. Examples of bodily secretions include cervical secretions, trachial-bronchial secretions, and pharyngeal secretions. Suitable bodily fluids include blood, tears, and central nervous system fluid. Examples of tissue specimens include a variety of biopsies, such as a biopsy of lung tissue or fallopian tube tissue. In addition, tissue from substantiate all areas may be examined within a postmortem examination, including heart tissue and lung tissue.

In accordance with the present invention, continuous hybrid cell lines are established that produce monoclonal antibodies directed against an antigenic determinant of the TWAR strain defined above defined in claim for use in the methods described above. In a particularly preferred embodiment, the cell line comprises the hybridoma clone RR 402 or TT 205. Monoclonal antibodies produced by such cell lines are also disclosed.

These and other aspects of the present invention will become evident upon reference to the following detailed description and attached drawings.

### Brief Description of the Drawings

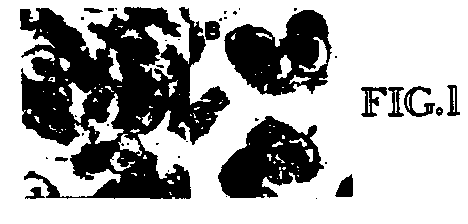

Figure 1A is a photomicrograph demonstrating TWAR inclusions morphologically typical of C. psittaci.
Figure 1B is a photomicrograph demonstrating the vacuolar structure typical of C. trachomatis.

### Best Mode for Carrying Out the Invention

As noted above, seroepidemiological studies have implicated an unusual strain of Chlamydia In several pneumonia epidemics in northern Europe and elsewhere. This new strain of Chlamydia is herein referred to as "TWAR," from the laboratory designations of the first two isolates (TW-183 and AR-39). TWAR has been shown to belong to the genus Chlamydia, but may be differentiated from C. trachomatis and C. psittaci by several criteria. With respect to inclusion morphology and presence of glycogen in inclusions, TWAR inclusions are more closely related to C. psittaci based upon their oval and dense appearance and absence of glycogen, in contrast to the vacuolar nature of C. trachomatis inclusions which contain glycogen. In addition, the TWAR organism does not react with C. trachomatis species-specific monoclonal antibody (Stephens et al., J. Immunol. 128:1083,1982).

While the TWAR organism clearly does not belong to the C. trachomatis species, it also exhibits important differences from currently recognized C. psittaci strains. TWAR and the other C. psittaci strains are immunologically distinct; no cross-reactions with the other C. psittaci strains have been found. Further, in the production of monoclonal antibodies against the TWAR organism (more thoroughly described below), no C. psittaci species-specific antibodies were detected, only genus- and strain- (TWAR serovar) specific antibodies were found. It is also interesting to note that while a plasmid has been found In almost all C. psittaci and C. trachomatis chlamydial strains examined, the TWAR organism has no plasmid. In addition, while all known C. psittaci strains have an animal or bird host, no animal or bird host for the TWAR organism has been found. In accordance with the present invention, the TWAR organism is therefore defined to include any Chlamydia strain that is morphologically similar to but distinct from C. psittaci, and immunologically distinct from currently recognized C. psittaci strains. A representative TWAR strain (AR-39) has been deposited with the American-type culture collection and assigned accession number 53592.

Hybridoma formation and production of monoclonal antibodies have been shown to be useful in immunodiagnosis of a variety of microbiological diseases. More specifically, monoclonal antibodies allow detection and serological classification of human infections. In addition, these monoclonal antibodies facilitate identification of genus-and species-specific antigens.

As noted above, continuous hybrid cell lines are established which are capable of producing monoclonal antibodies to an antigenic determinant of the TWAR organism. A general protocol for establishing such cell lines indudes immunizing an experimental animal, usually a mouse, with an appropriate antigen. Immune spleen calls are then isolated and fused with myeloma or lymphoma cells by exposure to a chemical agent, such as polyethylene glycol. Fused hybrid cells are then incubated in a selective medium. such as HAT medium, that precludes the growth of the malignant cell line. Hybridoma cells are cloned by limiting dilution, and culture supernatants are assayed for secretion of monoclonal antibody having a desired specificity. Large yields of selected monoclonal antibodies may be obtained through ascitic growth of hybridomas in vivo.

In order to obtain monoclonal antibodies that are specific for the TWAR organism, an appropriate TWAR antigen must be prepared. TWAR isolates may be cultured from throat (oropharynx) specimens of patients, either through eukaryotic cell culture or embryonated egg yolk sac culture. Most primary isolates require egg-adaptation of the TWAR organism prior to successful eukaryotic cell culture. In particular, it may be preferable to passage the TWAR isolates once or twice in yolk sac culture and then inoculate them onto cultures of HeLa 229 cells propagated in vials. It is also preferable to pretreat the HeLa cells with DEAE-dextran and cyclohexamide. The TWAR isolates may be adapted to grow in cell culture by repeated culture passages. In particular, approximately 10 or more serial culture passages may be advantageous for adaptation to HeLa cell growth.

Referring now to Figures 1A and 1B, photomicrographs are presented showing a HeLa 229 cell monolayer infected with a TWAR organism and a C. trachomatis, respectively. Figure 1A demonstrates TWAR inclusions morphologically typical of C. psittaci. The TWAR infection exhibits relatively dense cytoplasmic inclusions that have not indented or displaced the nucleus. In contrast, Figure 1 B demonstrates typical vacuolar inclusions of C. trachomatis that indent the nucleus.

The infected cell cultures are normally monitored for TWAR organism infectivity; and when a certain percentage of cells are infected, multiple vial cultures are inoculated in preparation for growth in large culture bottles. In a preferred embodiment, when 50%. infectivity is obtained, 20 to 30 vials of HeLa 229 cells may be infected to provide the inoculum for a 32-oz prescription bottle culture. Subsequent serial pasages will increase the number of TWAR strain Chlamydia. This adaptation and scale-up will yield a sufficient quantity of TWAR antigen for monoclonal antibody production and screening.

The immunogen for hybridoma production employed within the present invention was purified TWAR antigen. In a preferred embodiment, the antigen is Renografin-purified TWAR elementary bodies (>99% purified). Hybridomas may be prepared by fusion of immune mouse spleen cells with NS-0 myeloma cells, and hybridoma clones secreting anti-chlamydial antibodies identified. In a preferred embodiment, hybridoma culture supernatants are screened by ELISA. In a particularly preferred embodiment, the ELISA employs peroxidase-conjugated anti-mouse immunoglobulins and a colorimetric detection system. Further testing of monoclonal antibody specificity by a micro-immunofluorescence technique (S. P. Wang and J. T. Grayston, Amer. J. Ophthal. 70: 3678, 1970) is also preferred.

TWAR-elicited hybridomas may be intraperitoneally inoculated into syngeneic BALB/c mice. It is preferable to Pristane-prime the mice 2 weeks prior to inoculation. The resultant ascites fluid may be harvested, and monoclonal antibodies purified. In a preferred embodiment, ascitic monoclonal antibodies are affinity purified on a protein-A-sepharose column.

Selected TWAR monoclonal antibodies may be labeled for use within the methods described herein. Preferred labels include enzymes, fluorophores, radioisotopes, and luminescers. These and other labels are well known in the art. A particularly preferred label in this regard is fluorescein. The labeled TWAR monoclonal antibodies may then be examined for genus-, species- and strain-specificity. As shown in Table 1, when employed in a micro-immunofluorescence assay, the TWAR-elicited monoclonal antibodies reacted with TWAR isolates, but not C. trachomatis or C. psittaci.

**TABLE 1**

| Specificity of TWAR-Elicited Monoclonal Antibodies | | | |
|---|---|---|---|
| Chlamydial Strains | | Micro-IF Antibody Titer with TWAR-Elicited MAb | ELISA Antibody Titer with Genus-Specific MAb |
| TWAR- | TW 183 | 6400 | 3200 |
| | AR 39 | 6400 | 3200 |
| | AR 277 | 6400 | 3200 |
| | AR 388 | 6400 | 3200 |
| C. psittaci (6 strains) | | 0 | 3200 |
| C. trachomatis (15 serovars) | | 0 | 3200 |

Fluoroscein-conjugated TWAR monoclonal antibodies specfically stained TWAR elementary bodies and TWAR inclusion in infected cultured cells, but did not stain other Chlamydia. The present invention thus provides a monoclonal antibody that binds to a TWAR-specific antigenic determinant.

Within the present invention, a TWAR-specific monoclonal antibody has been employed in diagnostic assays of biological specimens. In particular, the present invention provides methods for detecting TWAR organisms in smears of clinical specimens and in inoculated cell cultures. These methods utilize labeled TWAR-specific monoclonal antibody that binds directly to TWAR-specific antigenic determinants in immobilized specimens. The specimens may be immobilized on a solid support, such as a glass slide, coverslip, or a microtiter well. The specimen or biological sample should also be fixed to the solid support, as by methanol or ethanol fixation. After the sample is fixed or bound to the surface, the sample is exposed to the labeled monoclonal antibody. Alternatively, a labeled second antibody capable of binding to the TWAR-specific monoclonal antibody may be employed. This modification of the method may provide an enhancement in the signal of the label. Identification of TWAR organisms in smear specimens by a direct fluorescent antibody technique is rapid, simple, and relatively inexpensive. The smear technique can be used both in clinical settings (early diagnosis, improved opportunity for isolation of the causative organism, follow-up on success of treatment) and in epidemiological settings.

In addition to providing a method for detecting the presence of the TWAR organism, the present invention provides a method for determining the presence of antibodies to the TWAR organism in biological samples. A particularly preferred embodiment for the screening of serum or bodily fluids for TWAR antibodies is the micro-IF assay. Typically, antigen (Chlamydia elementary body antigen) is fixed onto a microscopic slide, and the slide incubated with a sample suspected of containing TWAR antibody. A labeled specific binding partner is then added (such as fluorescein-conjugated anti-mouse immunoglobulin) and the reaction observed to determine the presence of antibodies to the TWAR organism.

The following examples are offered by way of illustration, and not by way of limitation.

### EXAMPLE 1

### Growth and Isolation of TWAR Organisms

### A. Cell Culture Isolation

Specimens of throat (oropharynx) swabs in transport media (sucrose/potassium/glutamate; SPG) from patients with acute respiratory disease were cultured according to a method modified from C. C. Kuo et al. (J. Infect. Dis. 125: 665-668, 1972). Each specimen was inoculated onto three vials containing HeLa 229 cell monolayers which had been pretreated with DEAE-dextran (30 Ög/ml) and cyclohexamide (0.5-0.8 Ög/ml) prior to inoculation. The inoculum was centrifuged onto the cells at 2,200 rpm for 60 minutes at room temperature. The vials were then incubated at 35°C for 72 hours (passage 1). The monolayer from one of the vials was fixed with methanol and stained with fluorescent antibody to search for inclusions indicative of infection. The other two vials were available to provide inoculum for a second passage.

### B. Egg Culture Isolation

Specimens of throat (oropharynx) swabs in SPG transport media from patients with acute respiratory disease were inoculated into the yolk sac of embryonated eggs, 6 to 8 days of age, according to the method of J. T. Grayston et al. (Proc. Soc. Exp. Biol. Med. 103: 596, 1960). Streptomycin was added to the specimen until a concentration was reached that gave 10 mg per egg on inoculation. The eggs were incubated at 35°C and candled daily. Upon death, or survival for 12 to 13 days, yolk sacs were harvested and stained by a modified Macchiavello staining method to look for elementary bodies of Chlamydia. When TWAR monoclonal antibody conjugated to fluorescein became available for staining the infected yolk sacs, the TWAR organisms could be seen much more readily. Several passages previously considered negative by Macchiavello staining were found to contain the TWAR organism. Positive cultures usually demonstrated increased numbers of elementary bodies on subsequent passage.

### C. Adaptation of TWAR Strains to Grow in Cell Cultures

All but one of the 13 TWAR isolates so far obtained required original inoculation in the yolk sac of embryonated eggs, with egg adaptation of the organisms (one or two passages) prior to cell culture inoculation to successfully adapt most of the TWAR isolates to HeLa 229 cell culture vials.

Serial passages of TWAR organisms were carried out in vial cultures of HeLa 229 cells, modified from the method of Kuo et al. (in "Nongonococcal Urethritis and Related Infections," pp. 328-336, 1977). As described for cell culture isolation (Example 1.A.), three separate HeLa cell monolayers in vials were inoculated for each passage. One was fixed and stained to determine the progress of the infection, and the remaining two monolayers were scraped from the vials and reinoculated exactly as described above for the first passage. At least 10 serial cell culture passages were required for adaptation.

When the infectivity increased so that greater than 50% of the cells were infected, the number of vials inoculated was increased to provide 20 to 30 vials of the infected cells for inoculation into a large culture bottle (32-oz prescription bottle). Serial passages were continued in the bottle culture until 100% of the cells were infected.

### EXAMPLE 2

### Preparation of TWAR Antigen

Following adaptation of a TWAR strain to 32-oz prescription bottles, the strain was expanded by growth in additional bottles of HeLa 229 cells. The organisms were harvested by rolling the monolayer with glass beads in 20 ml of Hanks balance salt solution. The cells were disrupted by sonication, and the HeLa cell debris was removed by low-speed centrifugation (1,200 rpm for 10 minutes). The organisms were then pelleted by high-speed centrifugation (16,000 rpm for 20 minutes). The TWAR elementary bodies were purified first by cushioning through a 30% Renografin suspension at 16,000 rpm for 40 minutes, which removes approximately 60% of the contaminants, and then by passing through a 30% to 65% Renografin linear gradient at 23,00 rpm for 90 minutes. The suspended band of TWAR elementary bodies was more than 99% purified. The method employed was modified from that of R. S. Stephens et al. (J. Immunol. 128: 1083, 1982).

### EXAMPLE 3

### Immunization Schedule

BALB/c mice were immunized with 5 x 10⁷ formalin-killed TWAR organisms, intraperitoneally on day 1 and with a similar inoculum intravenously at weekly intervals thereafter for 3 or 4 additional injections. The spleens were harvested 3 days after the last immunization.

### EXAMPLE 4

### Construction of Hybridomas

The NS-0 myeloma cell line was obtained from Milstein to be used for the fusion procedure. NS-0 cells were routinely grown in RPMI 1640 (GIBCO, Grand Island, N.Y.) containing 15% heat-inactivated fetal calf serum, 1 mM glutamine, and 1 mM pyruvate (referred to as "complete RPMI").

Spleen cell suspensions were prepared by mincing and passing the cells through a fine nylon screen. Lymphocyte cell suspensions were washed three times in serum-free RPMI; the NS-0 cells were washed once in serum-free RPMI.

NS-0 cells and lymphocytes were fused (at a 1:4 ratio or 1:8 ratio) in 40% polyethylene glycol by centrifugation at 250 x G for 10 minutes. The cells were washed with 10 vol of complete RMPI and centrifuged at 160 x G for 5 minutes. The supernatant was aspirated, and the cells were gently resuspended to 2.5 x 10⁶ cells/ml in complete RMPI supplemented with HAT medium (complete RMPI containing 1.0 x 10⁻⁴ M hypoxanthine, 4.0 x 10⁻⁷ M aminopterin, and 1.6 x 10⁻⁵ M thymidine). This hybrid cell suspension was mixed with thymocytes from 3- to 5-week-old BALB/c mice to a final concentration of 2.5 x 10⁶ thymocyltes/ml. The final cell suspension was seeded (in 200 Öl volumes) into the wells of 96-well microtest plates (Costar, Cambridge, Mass.). Additional feedings with HAT medium (50% substitution by volume) were given on days 5 and 8. The continued handling, feeding schedule, and cloning by limiting dilution were performed as described by Stephens et al. (supra).

### EXAMPLE 5

### Screening of Hybridoma Clones

Anti-chlamydial antibodies in culture fluids were detected by enzyme-linked immunosorbent assay (ELISA). For the antibody assay, 1 x 10⁵ Chlamydia organisms in 50 Öl of phosphate-buffered saline (PBS, pH 7.6) were adsorbed by the individual wells of a microtest plate by incubation overnight at 37° C. Control plates were prepared by allowing uninfected HeLa 229 cells (10⁴ cells/well) to attach to wells of microtiter plates. The following morning, the wells of the plates were blocked from further nonspecific protein adsorption by a 2-hour incubation with 75 Öl of 5% bovine serum albumin (BSA) in PBS. The antibody assay was performed in three steps:
(1) Fifty microliters of culture fluid were incubated in each of the antigen-adsorbed wells for 60 minutes at 37°C. Nonbound immunoglobulins were then removed from the wells by washing three times with PBS containing 1% BSA.
(2) Peroxidase-conjugated anti-mouse immunoglobulins (50 Öl) were added to each well and incubated for 60 minutes at 37° C. Residual unbound peroxidase conjugate was removed by washing three times with PBS.
(3) The immune reactions were detected by adding the substrate o-phenylenediamine and H₂O₂, and the color reaction quantitated.
Antibody specificity was further determined by testing the culture supernatants that were positive by ELISA in the micro-IF technique (see Example 6).

### EXAMPLE 6

### Production of Monoclonal Antibodies

Intraperitoneal inoculation of 1 to 5 x 10⁶ hybrid cells into syngeneic BALB/c mice induced palpable tumors (hybridomas) within 2 to 3 weeks. To assure tumor induction, syngeneic hosts were primed 2 weeks earlier by an intraperitoneal inoculation of 0.5 ml of Pristane (2, 6, 10, 14-tetramethyl pentadecane, Aldrich Chemical Co., Milwaukee, Wis.). In most instances (>90%), the progressive growth of the intraperitoneal transplanted hybridomas was accompanied by production of ascites fluid (1.0 to 5.0 ml/mouse).

Isotypes of the monoclonal antibodies contained in the ascites fluid were determined by ELISA using the MonoAb-Screen system (Zymed Laboratories, San Francisco, Calif.). Before use, ascites fluids were tested for specific antibody activity by the micro-IF technique.

For the micro-IF assay, culture fluids or ascites fluids were reacted with Chlamydia elementary body antigens which were placed on microscope slides for 30 minutes at 37°C in a moist chamber. After reaction, the slides were washed five times with PBS and air dried. Fluorescein-conjugated anti-mouse immunoglobulins were added and incubated for 30 minutes at 37°C in a moist chamber. After incubation, the slides were washed five times with distilled water and air dried. A drop of FA mounting fluid was added, and a coverslip was placed on the slide. The reaction was observed under FA-scope. For staining of inclusions, infected HeLa cells were fixed for 10 minutes in absolute methanol and assayed for TWAR-specific antibody activity.

Monoclonal antibodies contained within ascites fluid were affinity purified on a protein A-Sepharose column (Pharmacia Fine Chemicals, Uppsala, Sweden). Fluorescein isothiocyanate-conjugated monoclonal antibodies were prepared by the method of Goding (J. Immunol. Meth. 13: 215, 1976) and were used at an appropriate dilution as determined by titration for direct immunofluorescence assays. Unconjugated monoclonal antibodies were usually used at 1:100 dilution for indirect immunofluorescence techniques.

### EXAMPLE 7

### Diagnostic Assay

### A. Smear Technique

Monoclonal antibodies to C. trachomatis have previously been used for direct fluorescent antibody (FA) assay of smears from clinical specimens. Fluorescein-conjugated monoclonal antibodies specific for strains of C. psittaci have been employed in a similar direct FA assay of respiratory specimens. Briefly, a pharyngeal swab was rolled on a glass slide over two premarked circular areas (5 mm diameter), and the slide was then immersed in 95% ethanol for 10 to 30 minutes. After the slide dried in air, the fluorescent monoclonal antibody conjugate was applied to the specimen area. The slide was incubated at 37°C for 30 minutes in a moist chamber, washed by briefly dipping through six jars of distilled water, and dried. A drop of Bacto fluorescent antibody mounting fluid (Difco Laboratories, Detroit, Mich.) and a coverslip were applied to the slide, which was then examined under oil immersion at x 500 magnification with an epifluorescence microscope (Carl Zeiss, Oberkochen, West Germany). The entire area of the smear was examined. Findings on slides were considered positive for TWAR strain if at least five fluorescing elementary bodies were found in the specimen. Slides with negative findings were read for 5 minutes.

Throat swab smears from six of seven patients with TWAR isolations were stained with fluorescent TWAR monoclonal antibody. In four, typical elementary bodies of Chlamydia were demonstrated.

### B. Culture Technique

Fluorescein-conjugated TWAR strain-specific monoclonal antibody was used to identify TWAR isolates in initial HeLa 229 cell culture passage. The cell culture isolation technique is described in Example 1.A. Direct fluorescent antibody (FA) staining with TWAR strain-specific monoclonal antibody has greatly facilitated identification of TWAR isolates and has allowed direct passage of these isolates. Each of the TWAR isolates has been identified in primary HeLa 229 cell culture passage by direct FA staining of inclusions.

### C. Serological Technique

TWAR antigens, which were mixed with 3% normal yolk sac homogenate (to assist localization and attachment of the antigens on a slide), were dotted on a microscopic slide, air dried, fixed with acetone for 15 minutes, and air dried. Serial twofold dilutions of serum or bodily fluids (such as tears) were applied onto the antigens and incubated for 30 minutes at 30°C in a moist chamber. Within this assay, the fluids were reacted with Chlamydia elementary body antigens. After reaction, the slides were washed five times with PBS and air dried. Fluorescein-conjugated anti-mouse immunoglobulins were added and incubated for 30 minutes at 37°C in a moist chamber. After incubation, the slides were washed five times with distilled water and air dried. A drop of FA mounting fluid was added, and a coverslip was placed on the slide. The reaction was observed under FA-scope. For staining of inclusions, infected HeLa cells were fixed for 10 minutes in absolute methanol and assayed for TWAR-specific antibody activity.

Table 2 illustrates the high frequency of TWAR antibody present in the sera of adults from seven different areas of the world. Further, antibody was found to be present more frequently in males than in females.

**TABLE 2**

| | Denmark | Helsinki | | Nova Scotia |
|---|---|---|---|---|
| # Tested | 555 | 271 | | 200 |
| | Percent TWAR Antibody | | | |
| Male | 48 | 47 | | 35 |
| Female | 41 | 36 | | 18 |

| | Taiwan | Japan | Panama | Seattle |
|---|---|---|---|---|
| # Tested | 64 | 196 | 934 | 1042 |
| | Percent TWAR Antibody | | | |
| Male | 59 | 45 | - | 45 |
| Female | 52 | - | 40 | 36 |

## Claims

1. A monoclonal antibody directed against an antigenic determinant of a Chlamydial organism with accession number ATCC 53592 termed TWAR organism ATCC 53592 and which reacts with said organism but not with Chlamydia organisms other than those exhibiting the following characteristics:-
a) causes acute human respiratory disease ;
b) have no animal or bird host;
c) have cyto plasmic inclusions of oval and dense appearance In which glycogen is absent and which have not indented or displaced the nucleus;
d) are immunologically distinct from C psittaci strains;
e) do not react with Chlamydia trachomatis species-specific monoclonal antibody; and
f) contain no plasmid;

2. A continuous hybrid cell line that produces monoclonal antibody directed against an antigenic determinant of the TWAR strain of Chlamydia defined in claim 1.

3. cell line of Claim 2 wherein the cell line is a hybrid of an immune spleen cell capable of producing antibodies to the TWAR strain of Chlamydia defined in claim 1 and a myeloma cell.

4. A method for detecting the presence of the TWAR strain of Chlamydia defined in claim 1 in a biological sample, comprising: exposing a biological sample suspected of containing the TWAR strain of Chlamydia defined in claim 1 to a labeled monoclonal antibody according to Claim 1 and detecting the presence of immunocomplexes formed between the monoclonal antibody and the TWAR strain of Chlamydia defined in claim 1.

5. The method of claim 4 whersin said label is selected from the group consisting of enzymes, fluorophores, radioisotopes, and luminescers.

6. The method, of Claim 4 or 5 wherein the step of detecting is by enzyme reaction, fluorescence, luminescence emission, or radioactivity.

7. The method of Claim 4, 5 or 6 wherein the biological sample is selected from the group consisting of bodily secretions, bodily fluids, and tissue specimens.

8. A method for determining the presence of antibodies to the TWAR strain of Chlamydia defined in claim 1 in a biological sample, comprising: incubating the biological sample with elementary bodies of said TWAR organism to form a reaction mixture; and analyzing the reaction mixture to determine the presence of antibodies to the TWAR strain of Chlamydia defined in claim 1.

9. The method of Claim 8 wherein the step of analyzing comprises contacting the reaction mixture with a labeled specific binding partner for the antibody and detecting the label.

10. The method of Claim 9 wherein the specific binding partner is selected from the group consisting of goat anti-human IgM and goat anti-human IgG.

11. The method of Claim 9 or 10 wherein said label is selected from the group consisting of enzymes, fluorophores, radioisotopes and luminescers.

12. The method of any one of Claims 8 to 11 wherein the biological sample is selected from the group consisting of bodily secretions, bodily fluids and tissue specimens.

13. A method for detecting the presence of the TWAR strain of Chlamydia defined in claim 1 in a biological sample comprising: combining a biological sample suspected of containing the TWAR strain of Chlamydia defined in claim 1 with HeLa cells such that the TWAR strain defined in claim 1 becomes intracellular; incubating the HeLa cells containing the TWAR strain defined in claim 1 exposing the HeLa cells/TWAR strain defined in claim 1 to a labeled monoclonal antibody directed antigenic determinant of the TWAR strain defined in claim 1 and detecting the presence of immunocomplexes formed between the TWAR strain defined in claim 1 and the labeled monoclonal antibody.

14. The method of Claim 13 wherein the step of combining comprises inoculating the HeLa cells with the sample and subsequently centrifuging the inoculated sample onto the HeLa cells.

15. The method of Claim 13 or 14 wherein the step of exposing Includes fixing the monolayers with methanol.

16. The method of claims 13, 14 or 15 wherein the label is selected from the group consisting of enzymes, fluorophores, radioisotopes and luminescers.

17. The method of any one of Claims 13 to 16 wherein the step of detecting is by enzyme reaction, fluorescence, luminescence emission or radioactivity.

18. The method of any one of Claims 13 to 17 wherein the biological sample is selected from the group consisting of bodily secretions, bodily fluids and tissue specimens.

## Revendications

1. Anticorps monoclonal dirigé contre un déterminant antigénique d'un organisme chlamydial avec le numéro d'accession ATCC 53592 appelé organisme TWAR ATCC 53592 et qui réagit avec ledit organisme mais pas avec les organismes du genre Chlamydia distincts de ceux présentant les caractéristiques suivantes :
a) provoquent une maladie respiratoire humaine aiguë ;
b) ne possèdent pas d'hôte animal ou aviaire ;
c) comportent des inclusions cytoplasmiques d'apparence ovale et dense desquelles le glycogène est absent et qui n'ont pas dentelé ou déplacé le noyau ;
d) sont immunologiquement distincts des souches de *C*. *psittaci* ;
e) ne réagissent pas avec un anticorps monoclonal spécifique de l'espèce *Chlamydia trachomatis ;* et
f) ne contiennent aucun plasmide.

2. Lignée cellulaire hybride continue qui produit un anticorps monoclonal dirigé contre un déterminant antigénique de la souche TWAR de Chlamydia définie dans la revendication 1.

3. Lignée cellulaire selon la revendication 2 dans laquelle la lignée cellulaire est un hybride d'une cellule splénique immunitaire susceptible de produire des anticorps contre la souche TWAR de Chlamydia définie dans la revendication 1 et d'une cellule de myélome.

4. Procédé de détection de la présence de la souche TWAR de Chlamydia définie dans la revendication 1 dans un échantillon biologique, comprenant : l'exposition d'un échantillon biologique suspecté de contenir la souche TWAR de Chlamydia définie dans la revendication 1 à un anticorps monoclonal marqué selon la revendication 1 ; et la détection de la présence d'immunocomplexes formés entre l'anticorps monoclonal et la souche TWAR de Chlamydia définie dans la revendication 1.

5. Procédé selon la revendication 4 dans lequel ledit marqueur est choisi dans le groupe constitué par les enzymes, les fluorophores, les isotopes radioactifs et les composés luminescents.

6. Procédé selon la revendication 4 ou 5 dans lequel l'étape de détection se fait par réaction enzymatique, fluorescence, émission de luminescence ou radioactivité.

7. Procédé selon la revendication 4, 5 ou 6 dans lequel l'échantillon biologique est choisi dans le groupe constitué par les sécrétions corporelles, les liquides corporels et les échantillons de tissu.

8. Procédé de détermination de la présence d'anticorps contre la souche TWAR de Chlamydia définie dans la revendication 1 dans un échantillon biologique, comprenant : l'incubation de l'échantillon biologique avec des corps élémentaires dudit organisme TWAR pour former un mélange réactionnel ; et l'analyse du mélange réactionnel pour déterminer la présence d'anticorps contre la souche TWAR de Chlamydia définie dans la revendication 1.

9. Procédé selon la revendication 8 dans lequel l'étape d'analyse comprend la mise en contact du mélange réactionnel avec un partenaire de liaison spécifique marqué pour l'anticorps et la détection du marqueur.

10. Procédé selon la revendication 9 dans lequel le partenaire de liaison spécifique est choisi dans le groupe constitué par l'IgM anti-humaine de chèvre et l'IgG anti-humaine de chèvre.

11. Procédé selon la revendication 9 ou 10 dans lequel ledit marqueur est choisi dans le groupe constitué par les enzymes, les fluorophores, les isotopes radioactifs et les composés luminescents.

12. Procédé selon l'une quelconque des revendications 8 à 11 dans lequel l'échantillon biologique est choisi dans le groupe constitué par les sécrétions corporelles, les liquides corporels et les échantillons de tissu.

13. Procédé de détection de la présence de la souche TWAR de Chlamydia définie dans la revendication 1 dans un échantillon biologique, comprenant : la combinaison d'un échantillon biologique suspecté de contenir la souche TWAR de Chlamydia définie dans la revendication 1 avec des cellules HeLa de telle sorte que la souche TWAR définie dans la revendication 1 devienne intracellulaire ; l'incubation des cellules HeLa contenant la souche TWAR définie dans la revendication 1 ; l'exposition des cellules HeLa/ souche TWAR définie dans la revendication 1 à un anticorps monoclonal marqué dirigé contre un déterminant antigénique de la souche TWAR définie dans la revendication 1 ; et la détection de la présence d'immunocomplexes formés entre la souche TWAR définie dans la revendication 1 et l'anticorps monoclonal marqué.

14. Procédé selon la revendication 13 dans lequel l'étape de combinaison comprend l'inoculation des cellules HeLa avec l'échantillon et ensuite la centrifugation de l'échantillon inoculé sur les cellules HeLa.

15. Procédé selon la revendication 13 ou 14 dans lequel l'étape d'exposition inclut la fixation des monocouches avec du méthanol.

16. Procédé selon la revendication 13, 14 ou 15 dans lequel le marqueur est choisi dans le groupe constitué par les enzymes, les fluorophores, les isotopes radioactifs et les composés luminescents.

17. Procédé selon l'une quelconque des revendications 13 à 16 dans lequel l'étape de détection se fait par réaction enzymatique, fluorescence, émission de luminescence ou radioactivité.

18. Procédé selon l'une quelconque des revendications 13 à 17 dans lequel l'échantillon biologique est choisi dans le groupe constitué par les sécrétions corporelles, les liquides corporels et les échantillons de tissu.

## Patentansprüche

1. Ein monoklonaler Antikörper, der gerichtet ist gegen eine antigene Determinante eines Chlamydialen Organismus mit der Zugangsnummer ATCC 53592, bezeichnet als der TWAR Organismus ATCC 53592, und welcher mit besagtem Organismus reagiert, jedoch nicht mit Chlamydia-Organismen die anders sind als jene, die die folgenden Eigenschaften aufweisen: -
a) verursacht akute humane Atmungskrankheit;
b) haben keinen Tier- oder Vogelwirt;
c) haben zytoplasmatische Einschlüsse von ovalem und dichtem Aussehen, in denen Glykogen fehlt und die keinen eingedrückten oder verdrängten Zellkern aufweisen;
d) immunologisch verschieden von C psittaci-Stämmen sind;
e) nicht mit einem Chlamydia trachomatis Spezies-spezifischen monoklonalen Antikörper reagieren; und
f) kein Plasmid enthalten.

2. Eine kontinuierliche Hybridzelllinie, die einen monoklonalen Antikörper produziert, der gerichtet ist gegen eine antigene Determinante des in Anspruch 1 definierten TWAR-Stammes von Chlamydia.

3. Die Zelllinie nach Anspruch 2, wobei die Zelllinie ein Hybrid einer Immun-Milzzelle, die in der Lage ist, Antikörper zu dem in Anspruch 1 definierten TWAR-Stamm von Chlamydia zu produzieren, und einer Myelomzelle ist.

4. Ein Verfahren zum Nachweis des Vorhandenseins des in Anspruch 1 definierten TWAR-Stammes von Chlamydia in einer biologischen Probe, welches umfasst, dass eine biologische Probe, die unter dem Verdacht steht, dass sie den in Anspruch 1 definierten TWAR-Stamm von Chlamydia enthält, einem markierten monoklonalen Antikörper nach Anspruch 1 ausgesetzt wird und dass das Vorhandensein von zwischen dem monoklonalen Antikörper und dem in Anspruch 1 definierten TWAR-Stamm von Chlamydia ausgebildeten Immunkomplexen nachgewiesen wird.

5. Das Verfahren nach Anspruch 4, wobei besagte Markierung ausgewählt ist aus der Gruppe, die aus Enzymen, Fluorophoren, Radioisotopen und Lumineszeren besteht.

6. Das Verfahren nach Anspruch 4 oder 5, wobei der Nachweisschritt durch Enzymreaktion, Fluoreszenz, Lumineszenzemission oder Radioaktivität erfolgt.

7. Das Verfahren nach Anspruch 4, 5 oder 6, wobei die biologische Probe ausgewählt ist aus der Gruppe, die aus Körperabsonderungen, Körperflüssigkeiten und Gewebeproben besteht.

8. Ein Verfahren zur Bestimmung des Vorhandenseins von Antikörpern zu dem in Anspruch 1 definierten TWAR-Stamm von Chlamydia in einer biologischen Probe, welches umfasst, dass die biologische Probe mit Elementarkörperchen des besagten TWAR-Organismus inkubiert wird, um eine Reaktionsmischung zu bilden; und dass die Reaktionsmischung analysiert wird, um das Vorhandensein von Antikörpern zu dem in Anspruch 1 definierten TWAR-Stamm von Chlamydia zu bestimmen.

9. Das Verfahren nach Anspruch 8, wobei der Analyseschritt umfasst, dass die Reaktionsmischung mit einem markierten spezifischen Bindungspartner fiir den Antikörper in Kontakt gebracht wird und die Markierung nachgewiesen wird.

10. Das Verfahren nach Anspruch 9, wobei der spezifische Bindungspartner ausgewählt ist aus der Gruppe, die aus Ziegen-anti-Human-IgM und Ziegen-anti-Human-IgG besteht.

11. Das Verfahren nach Anspruch 9 oder 10, wobei besagte Markierung ausgewählt ist aus der Gruppe, die aus Enzymen, Fluorophoren, Radioisotopen und Lumineszeren besteht.

12. Das Verfahren nach einem der Ansprüche 8 bis 11, wobei die biologische Probe ausgewählt ist aus der Gruppe, die aus Körperabsonderungen, Körperflüssigkeiten und Gewebeproben besteht.

13. Ein Verfahren zum Nachweis des Vorhandenseins des in Anspruch 1 definierten TWAR-Stammes von Chlamydia in einer biologischen Probe, welches umfasst, dass eine biologische Probe, die unter dem Verdacht steht, dass sie den in Anspruch 1 definierten TWAR-Stamm von Chlamydia enthält, mit HeLa-Zellen in der Weise kombiniert wird, dass der in Anspruch 1 definierte TWAR-Stamm intrazellulär wird; dass die HeLa-Zellen, die den in Anspruch 1 definierten TWAR-Stamm enthalten, inkubiert werden; dass die HeLa-Zellen/TWAR-Stamm, definiert in Anspruch 1, einem markierten monoklonalen Antikörper ausgesetzt werden, der gegen eine antigene Determinante des in Anspruch 1 definierten TWAR-Stammes gerichtet ist und dass das Vorhandensein von zwischen dem in Anspruch 1 definierten TWAR-Stamm und dem markierten monoklonalen Antikörper gebildeten Immunkomplexen nachgewiesen wird.

14. Das Verfahren nach Anspruch 13, wobei der Kombinationsschritt Beimpfen der HeLa-Zellen mit der Probe und anschließendes Zentrifugieren der beimpften Probe auf die HeLa-Zellen umfasst.

15. Das Verfahren nach Anspruch 13 oder 14, wobei der Schritt des Aussetzens einschließt, dass die Monoschichten mit Methanol fixiert werden.

16. Das Verfahren nach Anspruch 13, 14 oder 15, wobei die Markierung ausgewählt ist aus der Gruppe, die aus Enzymen, Fluorophoren, Radioisotopen und Lumineszeren besteht.

17. Das Verfahren nach einem der Ansprüche 13 bis 16, wobei der Nachweisschritt durch Enzymreaktion, Fluoreszenz, Lumineszenzemission oder Radioaktivität erfolgt.

18. Das Verfahren nach einem der Ansprüche 13 bis 17, wobei die biologische Probe ausgewählt ist aus der Gruppe, die aus Körperabsonderungen, Körperflüssigkeiten und Gewebeproben besteht.
